(19) **Europäisches Patentamt / European Patent Office / Office européen des brevets**

(11) **EP 4 279 059 A1**

(12) # EUROPEAN PATENT APPLICATION
published in accordance with Art. 153(4) EPC

(43) Date of publication:
**22.11.2023 Bulletin 2023/47**

(21) Application number: **22912782.4**

(22) Date of filing: **31.03.2022**

(51) International Patent Classification (IPC):
*A61K 8/64* (2006.01)    *A61K 8/14* (2006.01)
*A61Q 19/00* (2006.01)    *A61K 9/00* (2006.01)
*A61K 47/42* (2017.01)    *A61K 9/127* (2006.01)
*C07K 7/08* (2006.01)

(86) International application number:
**PCT/KR2022/004657**

(87) International publication number:
**WO 2023/191148 (05.10.2023 Gazette 2023/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Cellicon Lab Inc.**
**Daejeon 34054 (KR)**

(72) Inventors:
• **SOUNG, Min Gyu**
**Yuseong-gu, Daejeon 34209 (KR)**

• **PARK, Bo Kyung**
**Seo-gu, Daejeon 35251 (KR)**
• **CHOI, Won Suk**
**Yuseong-gu, Daejeon 34189 (KR)**
• **JEONG, Young Pil**
**Yuseong-gu, Daejeon 34050 (KR)**
• **PARK, Jae Hoo**
**Yuseong-gu, Daejeon 34179 (KR)**
• **PARK, Eun Jeong**
**Dong-gu, Daejeon 34555 (KR)**

(74) Representative: **Zaccaro, Elisabetta et al**
**Notarbartolo & Gervasi S.p.A.**
**Viale Achille Papa, 30**
**20149 Milano (IT)**

(54) **NOVEL SKIN-PENETRATING PEPTIDE, DERMAL AND MUCOSAL COMPOSITION COMPRISING SAME, AND USE THEREOF**

(57) A skin-permeable peptide, a derivative thereof, or a fragment thereof of the present disclosure effectively delivers skin bioactive molecules deep into the skin tissues through assembly or fusion methods, thereby providing outstanding skin permeability as well as excellent skin residual effect of bioactive materials to maximize bioactive efficacy. Accordingly, the skin-permeable peptide, derivative thereof, or fragment thereof may be widely used as an active ingredient of a pharmaceutical composition for external use and a functional cosmetic composition that target skin tissues including mucous membranes.

[FIG. 2]

EP 4 279 059 A1

## Description

**[Technical Field]**

The present disclosure relates to a composition for transdermal delivery of a bioactive material, the composition including a skin-permeable peptide, a derivative thereof, or a fragment thereof; a skin-permeable composition; a carrier for transdermal delivery; a skin-permeable carrier; a pharmaceutical composition; a quasi-drug composition; a cosmetic composition; a kit including the composition for transdermal delivery; a method of preparing the composition for transdermal delivery, the cosmetic composition, and the quasi-drug composition; a transdermal delivery method of a bioactive material; and use for the transdermal delivery of a bioactive material.

**[Background Art]**

Skin, which occupies the largest part of the human body, is composed of the epidermis, dermis, and subcutaneous tissue, and performs various functions in the external environment. In particular, due to the unique characteristics of the stratum corneum constituting the skin barrier, it is known that a molecular weight of a skin-penetrating substance is about 500 Da or less. However, it is known that low-molecular-weight substances also have low skin permeation efficiency, and high-molecular-weight substances such as growth factors have even lower permeation efficiency. Therefore, in the cosmetics and pharmaceutical industries, there is a great need for safe methods for effective delivery of bioactive materials.

Physical delivery systems such as microneedles may cause irreversible damage to the skin. In the existing skin-permeable substances, lipophilic substances or polymers are often used (Korean Patent No. 10-2274435), and it is likely to cause various side effects and unknown toxicity in the human body. Thus, it is necessary to safely deliver bioactive materials to the dermis using peptides, which are fragments of proteins present in the human body.

**[Disclosure]**

**[Technical Problem]**

The present inventors completed the present invention by developing a skin-permeable peptide. The biomembrane penetrating peptide technology developed in the present disclosure is a state-of-the-art biotechnology that greatly improves the absorption rate of raw materials in the body and increases bioavailability, and is a next-generation new biomaterial platform technology that fully demonstrates its efficacy even if a trace amount is injected into raw materials of functional cosmetics or advanced medicines. Accordingly, the technology is intended for application to the development of skin-permeable cosmetics and medicines, *etc.*

**[Technical Solution]**

An object of the present disclosure is to provide a composition for transdermal delivery of a bioactive material, the composition including a skin-permeable peptide consisting of any one amino acid sequence of SEQ ID NO: 5 to SEQ ID NO: 8, a derivative thereof, or a fragment thereof.

Another object of the present disclosure is to provide a composition for a skin-permeation of bioactive material, the composition including the skin-permeable peptide, derivative thereof, or fragment thereof.

Still another object of the present disclosure is to provide a carrier for transdermal delivery of a bioactive material, the carrier including the skin-permeable peptide, derivative thereof, or fragment thereof.

Still another object of the present disclosure is to provide a skin-permeable carrier for a bioactive material, the carrier including the skin-permeable peptide, derivative thereof, or fragment thereof.

Still another object of the present disclosure is to provide a cosmetic composition including the composition for transdermal delivery.

Still another object of the present disclosure is to provide a quasi-drug composition including the composition for transdermal delivery.

Still another object of the present disclosure is to provide a pharmaceutical composition for external use on the skin or mucous membrane, the pharmaceutical composition including the composition for transdermal delivery.

Still another object of the present disclosure is to provide a kit including the composition for transdermal delivery and a bioactive material.

Still another object of the present disclosure is to provide a method of preparing the composition for transdermal delivery.

Still another object of the present disclosure is to provide a method of improving the skin using the composition for transdermal delivery.

Still another object of the present disclosure is to provide a method of preventing or treating a skin or mucosal disease using the pharmaceutical composition.

Still another object of the present disclosure is to provide a method of preparing the cosmetic composition and the quasi-drug composition.

Still another object of the present disclosure is to provide a transdermal delivery method of a bioactive material using the peptide consisting of any one amino acid sequence of SEQ ID NO: 5 to SEQ ID NO: 8, a derivative thereof, or a fragment thereof.

Still another object of the present disclosure is to provide use of the peptide consisting of any one amino acid sequence of SEQ ID NO: 5 to SEQ ID NO: 8, a derivative thereof, or a fragment thereof for the transdermal delivery of a bioactive material.

Still another object of the present disclosure is to provide use of the peptide consisting of any one amino acid sequence of SEQ ID NO: 5 to SEQ ID NO: 8, a derivative thereof, or a fragment thereof for the skin permeation of a bioactive material.

**[Advantageous Effects]**

A skin-permeable peptide, a derivative thereof, or a fragment thereof of the present disclosure effectively delivers skin bioactive molecules deep into the skin tissues through assembly or fusion methods, thereby providing outstanding skin permeability as well as excellent skin residual effect of bioactive materials to maximize bioactive efficacy. Accordingly, the skin-permeable peptide, derivative thereof, or fragment thereof may be widely used as an active ingredient of a pharmaceutical composition for external use and a functional cosmetic composition that target skin tissues including mucous membranes.

**[Brief Description of Drawings]**

FIG. 1 shows HPLC quantification of the result of a Franz diffusion cell assay to analyze skin permeation effects of peptides of SEQ ID NOS: 1 to 8 for a bioactive material, as compared to a known peptide;

FIG. 2 shows HPLC quantification of the result of a Franz diffusion cell assay over time to analyze the skin permeation effect of the skin-permeable peptide of the present disclosure for a bioactive material;

FIG. 3 shows fluorescence microplate reader quantification of the result of a Franz diffusion cell assay to analyze the skin permeation effect of the skin-permeable peptide of the present disclosure for bioactive materials with different molecular weights;

FIG. 4 shows fluorescence microplate reader quantification of the result of a Franz diffusion cell assay to analyze the skin permeation effect of the skin-permeable peptide of the present disclosure for a liposome; and

FIG. 5 shows the result of evaluating efficacy improvement according to the skin permeation effect of the skin-permeable peptide of the present disclosure for a bioactive material.

**[Detailed Description of the Invention]**

Hereinafter, the present disclosure will be described in detail as follows. Meanwhile, each description and embodiment disclosed in this disclosure may also be applied to other descriptions and embodiments. Further, all combinations of various elements disclosed in this disclosure fall within the scope of the present disclosure. Further, the scope of the present disclosure is not limited by the specific description described below.

An aspect of the present disclosure provides a composition for transdermal delivery of a bioactive material, the composition including a skin-permeable peptide consisting of any one amino acid sequence of SEQ ID NO: 5 to SEQ ID NO: 8, a derivative thereof, or a fragment thereof.

As used herein, the term "skin permeation" means biomembrane permeation, and the "skin-permeable peptide" means a peptide having a biomembrane permeability. The skin-permeable peptide may have cell permeability and biomembrane permeability, specifically, skin and/or mucosal permeability, but is not limited thereto.

The skin-permeable peptide of the present disclosure may include the skin-permeable peptide consisting of any one amino acid sequence of SEQ ID NO: 5 to SEQ ID NO: 8, a derivative thereof, or a fragment thereof.

The present disclosure may efficiently introduce, into cells and skin, bioactive molecules, which are difficult to be delivered through the skin due to the size of molecular weight or the unique characteristics of the stratum corneum of the skin.

As compared to existing skin-permeable peptides, the skin-permeable peptide of the present disclosure may deliver a bioactive material into skin cells or the dermal layer of the skin through a composition for transdermal delivery with improved cell and skin permeability, the composition including a skin bioactive molecule, a transdermal delivery system, and a skin-permeable peptide assembling or fusion method.

The skin-permeable peptide, a derivative thereof, or a fragment thereof of the present disclosure effectively delivers a skin bioactive molecule deep into the skin tissue through an assembling or fusion method to exhibit excellent skin permeability as well as excellent skin residual effect, thereby maximizing physiological activity. Accordingly, the present disclosure may be used as a component of a composition that targets skin tissues including mucous membranes.

In exemplary embodiments of the present disclosure, SEQ ID NO: 5 to SEQ ID NO: 8 and derivatives thereof were confirmed to have remarkable skin permeability (Examples 1 to 6).

As used herein, the term "derivative" means a derivative of the skin-permeable peptide consisting of any one amino acid sequence of SEQ ID NO: 5 to SEQ ID NO: 8, specifically, it may be a peptide having an amino acid sequence which is prepared by adding some functional groups or by deleting, modifying, substituting, or adding some amino acid sequence in the amino acid sequences of SEQ ID NO: 5 to SEQ ID NO: 8, but is not limited thereto.

Specifically, in the present disclosure, the derivative may be any one or more selected from the group consisting of SEQ ID NO: 1 to SEQ ID NO: 4, but is not limited thereto.

Specifically, the skin-permeable peptide consisting of any amino acid sequence of SEQ ID NO: 5 to SEQ ID NO: 8 of the present disclosure and the derivative thereof may have a sequence as in the following Chemical Formula 1 while having skin permeability, but is not limited thereto.

[Chemical Formula 1]     [X1-X2-Lys-Phe-X3-X4-Ile-Leu-X5-Tyr-Leu-X6]

In Chemical Formula 1, X1 to X6 may be selected from basic amino acids, and may be selected from arginine, lysine, leucine, and phenylalanine. Preferably, the skin-permeable peptide consisting of any one amino acid sequence of SEQ ID NO: 5 to SEQ ID NO: 8 of the present disclosure and the derivative thereof may have the sequence of Chemical Formula 1, and the derivative of the present disclosure may consist of an amino acid sequence of SEQ ID NO: 1 to SEQ ID NO: 4, but is not limited thereto.

SEQ ID NO: 1: $H_2N$–[Lys–Lys–Leu–Phe–Lys–Lys–Ile–Leu–Lys–Tyr–Leu–Lys]–$CO_2H$,

SEQ ID NO: 2: $H_2N$–[Arg–Arg–Leu–Phe–Arg–Arg–Ile–Leu–Arg–Tyr–Leu–Arg]–$CO_2H$,

SEQ ID NO: 3: $H_2N$–[Arg–Phe–Leu–Phe–Arg–Leu–Ile–Leu–Arg–Tyr–Leu–Arg]–$CO_2H$,

SEQ ID NO: 4: $H_2N$–[Arg–Arg–Leu–Phe–Arg–Leu–Ile–Leu–Arg–Tyr–Leu–Phe]–$CO_2H$,

SEQ ID NO: 5: $H_2N$–[Arg–Lys–Leu–Phe–Lys–Arg–Ile–Leu–Arg–Tyr–Leu–Lys]–$CO_2H$,

SEQ ID NO: 6: $H_2N$–[Lys–Arg–Leu–Phe–Arg–Lys–Ile–Leu–Lys–Tyr–Leu–Arg]–$CO_2H$,

SEQ ID NO: 7: $H_2N$–[Lys–Arg–Leu–Phe–Lys–Arg–Ile–Leu–Arg–Tyr–Leu–Lys]–$CO_2H$,

and

SEQ ID NO: 8: $H_2N$–[Arg–Lys–Leu–Phe–Arg–Lys–Ile–Leu–Lys–Tyr–Leu–Arg]–$CO_2H$

Specifically, the derivative may be a derivative in which an amino acid corresponding to any one position selected from the group consisting of positions 1, 2, 5, 6, 9, and 12 from the N-terminus of the skin-permeable peptide consisting of any one amino acid sequence of SEQ ID NO: 5 to SEQ ID NO: 8 is substituted with another basic amino acid.

The amino acid substitution may generally occur based on the polarity, charge, solubility, hydrophobicity, hydrophilicity, and/or amphipathic nature of residues. Specifically, among amino acids having electrically charged side chains (electrically charged amino acids), positively charged (basic) amino acids include arginine (R), lysine (K), and histidine (H), negatively charged (acidic) amino acids include glutamate (E) and aspartate (D); among amino acids having uncharged side chains (uncharged amino acids), nonpolar amino acids include glycine (G), alanine (A), valine (V), leucine (L), isoleucine (I), methionine (M), phenylalanine (F), tryptophan (W), and proline (P), and polar or hydrophilic amino acids include serine (S), threonine (T), cysteine (C), tyrosine (Y), asparagine (N) and glutamine (Q), and among the nonpolar amino acids, aromatic amino acids include phenylalanine, tryptophan, and tyrosine.

In the present disclosure, 'substitution with another basic amino acid' is not limited, as long as it is a substitution with a basic amino acid different from the amino acid before substitution. In other words, an amino acid before substitution, which is at any one position selected from the group consisting of positions 1, 2, 5, 6, 9, and 12 from the N-terminus of the skin-permeable peptide consisting of any one amino acid sequence of SEQ ID NO: 5 to SEQ ID NO: 8, may be substituted with another basic amino acid, specifically, an amino acid which is at any one position selected from the group consisting of positions X1, X2, X3, X4, X5, and X6 may be substituted with another basic amino acid. Specifically, the basic amino acid may be selected from histidine, lysine, and arginine, and may be lysine or arginine, but is not limited thereto.

With respect to the objects of the present disclosure, the derivative of the skin-permeable peptide consisting of any one amino acid sequence of SEQ ID NO: 5 to SEQ ID NO: 8 may be any one or more selected from the group consisting of SEQ ID NO: 1 to SEQ ID NO: 4, but is not limited thereto.

As used herein, the "fragment" refers to a partial sequence of a peptide having a specific sequence. The fragment of the present disclosure may be a partial sequence of the skin-permeable peptide consisting of any one amino acid sequence of SEQ ID NO: 5 to SEQ ID NO: 8 or a derivative thereof, wherein the partial sequence may also have the skin permeability like the skin-permeable peptide or the derivative thereof.

The skin-permeable peptide consisting of any one amino acid sequence of SEQ ID NO: 5 to SEQ ID NO: 8, a derivative thereof, or a fragment thereof may be derived from a natural product, but is not limited thereto, and any sequence may be included as long as it has the same activity as the skin-permeable peptide, derivative thereof, or fragment thereof.

Although the skin-permeable peptide of the present disclosure is described as the skin-permeable peptide consisting of any one amino acid sequence of SEQ ID NO: 5 to SEQ ID NO: 8, a derivative thereof, or a fragment thereof, a meaningless sequence addition upstream or downstream of the amino acid sequence of the skin-permeable peptide consisting of any one amino acid sequence of SEQ ID NO: 5 to SEQ ID NO: 8, a derivative thereof, or a fragment thereof, or a silent mutation that allows to retain the same function as the skin-permeable peptide is not excluded. It is obvious to those skilled in the art that as long as a peptide has an activity the same as or corresponding to the skin-permeable peptide consisting of any one amino acid sequence of SEQ ID NO: 5 to SEQ ID NO: 8, a derivative thereof, or a fragment thereof, it belongs to the skin-permeable peptide of the present disclosure. For specific example, the skin-permeable peptide of the present disclosure may be the skin-permeable peptide consisting of any one amino acid sequence of SEQ ID NO: 5 to SEQ ID NO: 8, a derivative thereof, or a fragment thereof, or a peptide having an amino acid sequence having 80%, 90%, 95%, 96%, 97%, 98%, or 99% or more homology or identity.

Even though a 'peptide including an amino acid sequence listed with a specific sequence number', a 'peptide consisting of an amino acid sequence listed with a specific sequence number', or a 'peptide having an amino acid sequence listed with a specific sequence number' is described in the present disclosure, as long as a peptide has an activity the same as or equivalent to that of the peptide consisting of the amino acid sequence of the corresponding sequence number, it is apparent that peptides having an amino acid sequence which is partially deleted, modified, substituted, conservatively substituted, or added may also be used in the present disclosure, for example, addition of a sequence which does not modify a function of the peptide at the N-terminus and/or C-terminus of the amino acid sequence, naturally occurring mutation, silent mutation thereof, or conservative substitution.

The "conservative substitution" means substitution of one amino acid with another amino acid that has similar structural and/or chemical properties. Such amino acid substitutions may generally occur on the basis of similarity in polarity, charge, solubility, hydrophobicity, hydrophilicity, and/or the amphipathic nature of residues. Commonly, conservative substitution has little or no effect on the activity of the peptide.

The peptide of the present disclosure may be prepared by consecutively forming amide bonds of one or more amino acids or appropriately protected amino acids on an amino acid backbone regularly bound to a solid phase, but is not limited thereto. Insertion, substitution, or deletion of another amino acid is also possible in the peptide within a range that does not significantly deteriorate stability, and this also falls within the scope of the present disclosure.

In addition, the skin-permeable peptide of the present disclosure may be prepared in the form of additionally coupling, to the C-terminus or the N-terminus, a known cell permeable peptide that promotes intracellular migration. For example, the known cell-permeable peptides may be a TAT peptide (Arg-Lys-Lys-Arg-Arg-Tyr-Arg-Arg-Arg) and a Tat-PTD peptide (Gly-Arg-Lys-Lys-Arg-Arg-Gln-Arg-Arg-Arg: Tat PTD), but the present disclosure is not limited thereto, and any cell-permeable peptide known in the art may be used as long as the permeable peptide does not inhibit the activity of the present disclosure.

The peptides and compounds of the present disclosure may be prepared in the form of metal complex compounds, wherein the metal may be selected from copper, magnesium, calcium, iron, zinc, nickel, silver, germanium, and gallium, but is not limited thereto. Preferably, copper may be used, but is not limited thereto.

Meanwhile, the peptides of the present disclosure may exist in the form of salts. The form of the salt usable in the present disclosure may be prepared during the final isolation and purification of the compounds or by reacting an amino group with a suitable acid. For example, acid addition salts may include acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, formate, fumarate, hydrochloride, hydrobromide, hydroiodide, 2-hydroxyethanesulfonate, lactate, maleate, mesitylenesulfonate, methanesulfonate, naphthylenesulfonate, nicotinate, 2-naphthalenesulfonate, oxalate, palmoate, pectinate, persulfate, 3-phenylpropionate, picrate, pivalate, propionate, succinate, tartrate, trichloroacetate, trifluoroacetate, phosphate, glutamate, bicarbonate, para-toluenesulfonate, and undecanoate, but are not limited thereto. In addition, examples of acids which may be employed to form acid addition salts may include inorganic acids such as hydrochloric acid, hydrobromic acid, sulfuric acid, and phosphoric acid, and organic acids such as oxalic acid, maleic acid, succinic acid, and citric acid, but are not limited thereto. Preferably, the peptide of the present disclosure may be prepared in the form of a trifluoroacetate salt or an acetate salt.

An amino group or carboxyl group of an amino acid, which is used for preparing the peptide included in the composition of the present disclosure, may be protected by a suitable protecting group. The protected amino acid may be attached to a solid support or reacted in a solution by adding the next amino acid under conditions suitable for forming the amide linkage. Alternatively, the protecting group may be completely removed prior to adding the amino acid protected with a suitable protecting group. After all amino acids have been linked as desired, the final target peptide may be obtained by sequentially or concurrently separating from the free residual protecting groups and the free solid support.

As the most preferred synthesis method of preparing the peptide compound of the present disclosure, a solid phase peptide synthesis method using a solid-phase polymer support may be used, and the $\alpha$-amino group of the peptide prepared by the above method may be protected by an acidic or basic sensitive functional group. At this time, the protecting group of the amino acid must have a stable property under the peptide condensation reaction conditions, and must have a property of being easily removable without destroying the growing peptide chain or without racemization of any of the chiral centers contained therein. Therefore, suitable protecting groups may include 9-fluorenylmethyloxycarbonyl (Fmoc), t-butoxycarbonyl (Boc), benzyloxycarbonyl (Cbz), biphenylisopropyl-oxycarbonyl, t-amyloxycarbonyl, isobornyloxycarbonyl, $(\alpha,\alpha)$-dimethyl-3,5-dimethoxybenzyloxycarbonyl, O-nitrophenylsulfenyl, 2-cyano-t-butyloxycarbonyl, *etc.* Other suitable protecting groups known in the art for this purpose may also be used within the scope of the present disclosure.

A 9-fluorenylmethyloxycarbonyl (Fmoc) protecting group may be used as the most preferred protecting group for amino acids used in the peptide synthesis of the present disclosure.

Particularly, as the protecting groups of the amino acid residues used in the peptide synthesis of the present disclosure, t-butyl (t-Bu) for *N*-methylglutamic acid; t-butoxycarbonyl (Boc) for lysine; 7t-butyl (t-Bu) for serine; t-butyl (t-Bu) for threonine and allothreonine; and trityl (Trt) for cysteine are preferred, but the present disclosure is not limited thereto.

In the solid phase peptide synthesis method, the C-terminal amino acid may be attached to a suitable solid support or resin. Suitable solid supports useful for the above synthesis are those materials which are inert to the reagents and reaction conditions of the stepwise condensation-deprotection reactions, as well as being insoluble in a medium to be used, for example, a 2-chlorotrityl chloride resin, rink amide, or rink amide 4-methylbenzylhydrylamine resin (rink amid MBHA resin).

In particular, the preferred solid support for the C-terminal peptides may be 2-chlorotrityl chloride, rink amide, or rink amide 4-methylbenzylhydrylamine resin (rink amide MBHA resin) available from Novabiochem Corporation.

The C-terminal amide may be condensed (bound, coupled) to the resin or the solid-phase support through condensation by activating carboxylic acid by $N,N'$-dicyclohexylcarbodiimide (DCC), $N,N'$-diisopropylcarbodiimide (DIC), [O-(7-azabenzotriazol-1-yl)-1,1,3,3-tetramethyluronium hexafluorophosphate] (HATU), or O-benzotriazol-1-yl-$N,N,N',N'$-tetramethyluroniumhexafluorophosphate (HBTU), in the presence or absence of 4-dimethylaminopyridine (DMAP), 1-hydroxybenzotriazole (HOBt), $N$-methylmorpholine (NMM), benzotriazol-1-yloxy-tris(dimethylamino)phosphonium-hexafluorophosphate (BOP) or bis(2-oxo-3-oxazolidinyl)phosphine chloride (BOPCI), for about 1 hour to about 24 hours under a temperature condition of 10°C to 50°C, preferably, under a temperature condition of 30°C in a solvent such as dichloromethane, $N$-methylpyrrolidone (NMP) or DMF.

When the solid support is a rink amide 4-methylbenzylhydrylamine resin, the Fmoc functional group as a preferred protecting group is cleaved with a secondary amine, preferably, 20% piperidine DMF solution in an excessive amount, prior to coupling with the C-terminal amino acid. The preferred reagents used in the coupling of the target amino acid to the deprotected 4-(2',4'-dimethoxyphenyl-Fmoc-aminomethyl)phenoxyacetamidoethyl resin are coupling reagents such as $N$-methylmorpholine (NMM), 1-hydroxybenzotriazole (HOBt), [O-(7-azabenzotriazol-1-yl)-1,1,3,3-tetramethyluronium hexafluorophosphate] (HATU), O-benzotriazol-1-yl-$N,N,N',N'$-tetramethyluroniumhexafluorophosphate (HBTU), $N,N'$-dicyclohexylcarbodiimide (DCC), or $N,N'$-diisopropylcarbodiimide (DIC) in a DMF solvent for the appropriately protected amino acid.

The coupling of successive amino acids carried out in the present disclosure may be carried out using an automatic polypeptide synthesizer widely known in the related art, or may be directly carried out manually. As preferred conditions for the synthesis reaction, $\alpha$-amino acid protected with the Fmoc group is deprotected by treatment with a secondary amine solution, preferably, piperidine, and then washed with a sufficiently excessive amount of solvent, and each protected amino acid to be coupled is then introduced in about 3- to 7-fold molar excess, and the reaction may be preferably carried out in the DMF solvent.

In the last step of the peptide synthesis using the solid-phase resin of the present disclosure, peptides to be obtained may be removed from the resin by performing manipulation consecutively or once, and protecting groups that protect amino acid residues may be deprotected. As conditions for removing the peptide from the resin and deprotecting the protecting groups present in the residues, a cleavage reagent cocktail that cleaves the resin-peptide bond, for example, a dichloromethane mixed cocktail solution containing trifluoroacetic acid (TFA), triisopropylsilane (TIS), thioanisole, water, or ethanedithiol (EDT), *etc.* may be generally used. The mixed solution thus obtained may form a precipitate by treating with an excessive amount of the refrigerated diethyl ether solvent. The precipitate thus obtained is completely precipitated by centrifugation, and the excess trifluoroacetic acid, triisopropylsilane, thioanisole, water, and ethanedithiol are primarily removed, and the above procedure is repeated twice or more to obtain a solidified precipitate. At this time, the completely deprotected peptide salts may be separated and purified using a mixed solvent consisting of water and acetonitrile solvent and reverse-phase high-performance liquid chromatography (HPLC). Solid peptides may be obtained by completely concentrating and drying the separated and purified peptide solution using lyophilization.

The present disclosure provides a composition for transdermal delivery of a bioactive material.

As used herein, the "bioactive material" is a concept that collectively refers to materials having physiological actions in the body. The bioactive material may be hydrophilic, hydrophobic, or poorly soluble, and any one is included without limitation as long as it may have a physiological action, such as natural products, chemical compounds, proteins, peptides, amino acids, nucleic acids, lipids, liposomes, polymers, *etc.* In addition, the bioactive material of the present disclosure may be used alone, and may include, but is not limited to, combinations with other bioactive materials, combinations with carriers, and combinations with known skin-permeable peptides.

In one embodiment, the bioactive material of the present disclosure may be a major component of medicines, quasi-drugs, and/or cosmetics used for skin, skin transdermal, and/or mucous membranes.

The bioactive material of the present disclosure may have one or more physiological actions, and may have physiological actions such as skin improvement, health promotion, disease prevention, improvement, *etc.*, but any one may be included without limitation as long as it may be used together with the skin-permeable peptide, derivative thereof, or fragment thereof of the present disclosure.

In one embodiment, the bioactive material may be any one or more selected from the group consisting of lidocaine, caffeine, collagen, hyaluronic acid, and liposome, but is not limited thereto.

The skin-permeable peptide consisting of any one amino acid sequence of SEQ ID NO: 5 to SEQ ID NO: 8, a derivative thereof, or a fragment thereof may be directly or indirectly linked to the bioactive material, but is not limited thereto.

In one embodiment, the skin-permeable peptide consisting of any one amino acid sequence of SEQ ID NO: 5 to SEQ ID NO: 8, a derivative thereof, or a fragment thereof and the bioactive material are directly linked to each other or linked via a linker, or may additionally include other protein moieties, but are not limited thereto. As the linkage method of the present disclosure, any method carried out in the art may be used without limitation, as long as the method does not change the structure or activity of the skin-permeable peptide consisting of any one amino acid sequence of SEQ ID NO: 5 to SEQ ID NO: 8, a derivative thereof, or a fragment thereof and the bioactive material to be linked. The linker may be a peptide linker consisting of 1 amino acid to 20 amino acids, or a non-peptide linker, but is not limited thereto.

In one embodiment, the skin-permeable peptide consisting of any one amino acid sequence of SEQ ID NO: 5 to SEQ ID NO: 8, a derivative thereof, or a fragment thereof of the present disclosure may be linked to the bioactive material via noncovalent bonds (*e.g.*, ionic bonds, hydrogen bonds, van der Waals bonds, *etc.*), but is not limited thereto.

In one embodiment, the skin-permeable peptide consisting of any one amino acid sequence of SEQ ID NO: 5 to SEQ ID NO: 8, a derivative thereof, or a fragment thereof of the present disclosure may be linked to the bioactive material (*e.g.*, via a covalent bond), but is not limited thereto. The skin-permeable peptide, a derivative thereof, or a fragment thereof and the bioactive material may be directly linked to each other, or may be linked via a linker, or may additionally include other protein moieties, but are not limited thereto.

In one embodiment, the skin-permeable peptide may perform self-assembling or self-fusion with the bioactive material.

In one embodiment, one or more molecules of the skin-permeable peptide may surround or fuse with the bioactive material, but is not limited thereto.

As used herein, the "transdermal delivery" means the delivery of a bioactive material through a biological membrane. The composition for transdermal delivery of the present disclosure may be for delivery of a bioactive material to cells and biomembranes, specifically, for delivery through skin and/or mucous membranes, but is not limited thereto.

The composition for transdermal delivery of the present disclosure may increase permeability of a bioactive material to any one or more selected from the group consisting of the skin and mucous membrane, but is not limited thereto.

The composition for transdermal delivery of the present disclosure may be used by further including a carrier, an excipient, a diluent, an antioxidant, and/or a buffer, *etc.,* as needed, but is not limited thereto.

In one embodiment, the carrier may be a liposome, but is not limited thereto.

As used herein, the "liposome" refers to a hollow structure formed by a phospholipid bilayer in an aqueous solution. The liposome may include a bioactive material inside a hollow structure, but is not limited thereto.

In one embodiment, the liposome may increase the skin permeability and/or transdermal delivery of a bioactive material by the skin-permeable peptide consisting of any one amino acid sequence of SEQ ID NO: 5 to SEQ ID NO: 8, a derivative thereof, or a fragment thereof of the present disclosure, but is not limited thereto.

In one embodiment of the present disclosure, the present disclosure confirmed that the skin permeability and transdermal delivery of a bioactive material of lidocaine, caffeine, collagen of 3 kDa, 30 kDa, and 300 kDa, and hyaluronic acid (HA) of 800 kDa may be increased, regardless of properties, size, and type thereof. Further, in one embodiment of the present disclosure, it was also confirmed that the skin-permeable peptide of the present disclosure increases skin permeability and transdermal delivery activity for liposomes (Examples 1 to 6). This suggests that the skin-permeable peptide of the present disclosure increases the skin permeability and transdermal delivery activity of liposomes containing bioactive materials.

Another aspect of the present disclosure provides a composition for skin-permeation of a bioactive material, the composition including the skin-permeable peptide consisting of any one amino acid sequence of SEQ ID NO: 5 to SEQ ID NO: 8, a derivative thereof, or a fragment thereof.

Any one amino acid sequence of SEQ ID NO: 5 to SEQ ID NO: 8, skin-permeable peptide, derivative, or fragment, bioactive material, and skin permeation are as described above.

In one embodiment, the composition for skin-permeation of the present disclosure may increase permeability of the bioactive material to any one or more selected from the group consisting of skin and mucous membrane, but is not limited thereto.

The composition for skin-permeation of the present disclosure may be used by further including a carrier, an excipient, a diluent, an antioxidant, and/or a buffer, *etc.,* as needed, but is not limited thereto.

Still another aspect of the present disclosure provides a carrier for transdermal delivery of a bioactive material, the carrier including the skin-permeable peptide consisting of any one amino acid sequence of SEQ ID NO: 5 to SEQ ID NO: 8, a derivative thereof, or a fragment thereof.

Still another aspect of the present disclosure provides a skin-permeable carrier for a bioactive material, the carrier including the skin-permeable peptide consisting of any one amino acid sequence of SEQ ID NO: 5 to SEQ ID NO: 8, a derivative thereof, or a fragment thereof.

Any one amino acid sequence of SEQ ID NO: 5 to SEQ ID NO: 8, skin-permeable peptide, derivative, fragment, bioactive material, transdermal delivery, and skin permeation are as described above.

As used herein, the "carrier" means those that are able to support any material or component, exemplified by the composition, and may be used interchangeably with a support, an impregnating agent, or a medium, *etc.* In addition, the composition, which is an example of the carrier, may be applied and delivered to the skin through an application means such as a hand, puff, tip, or brush, *etc.*

The composition for transdermal delivery, composition for skin-permeation, carrier for transdermal delivery, and skin-permeable carrier may be included in a pharmaceutical composition, quasi-drug composition, or cosmetic composition, *etc.,* but are not limited thereto.

With respect to the object of the present disclosure, the carrier for transdermal delivery of a bioactive material and the skin-permeable carrier may include the skin-permeable peptide consisting of any one amino acid sequence of SEQ ID NO: 5 to SEQ ID NO: 8, a derivative thereof, or a fragment thereof.

In one embodiment, the composition for transdermal delivery, composition for skin-permeation, carrier for transdermal delivery, and skin-permeable carrier of the present disclosure may be a skin preparation for external use.

As used herein, the "skin preparation for external use" means a formulation applied to the skin or mucous membrane. In the present disclosure, the preparation for external use is not limited to the shape, *etc.,* as long as it is applied to the skin or mucous membrane, and the preparation for external use may be further classified into ointments, creams, lotions, gels, *etc.,* but is not limited thereto.

Specifically, the ointments may be semi-solid preparations for external use, in which the main ingredient applied to the skin or mucous membrane is dissolved or dispersed in a base. The base may be divided into lipophilic ointments and water-soluble ointments according to their ingredients. Ointments using a lipophilic base may soften the skin or mucous membrane, have excellent sealability and are suitable for application to wounds, but have a disadvantage of not being easy to remove. The ointments may be formulations which are advantageous upon locally permeating ingredients that are difficult to permeate the skin, due to their lipophilic property similar to the skin or mucous membrane and skin softening action.

Since the ointments are mainly composed of an oily phase such as oil, they have excellent moisturizing properties and is good for permeation of lipophilic components, but has a disadvantage of being sticky. The creams may be a semi-solid external agent emulsified in a water-in-oil type, and may be a formulation of which stickiness is improved by mixing an oily phase and an aqueous phase. As an emulsifying process, a surfactant or a special process may be used to mix the oily phase and the aqueous phase. In addition, the creams may be formulations that are easier to apply and remove than the ointments, and the lotions are formulations having properties more similar to water than creams, and are preparations for external use, in which the main ingredient is dissolved, emulsified or finely dispersed in an aqueous liquid formulation. The main ingredient, additives, and purified water, *etc.* are used and all homogenized to prepare solutions, suspensions, or emulsions. The gels may be semi-solid preparations for external use, composed of organic molecules having a high molecular weight which are penetrated by liquid. The gels may be a dispersion of a gelling agent in water, and gelling agents such as carbomers or synthetic polymers may be used. Various types of preparations for external use may be used depending on the active ingredient, purpose of use, and method of use, etc., and the preparation for external use according to the present disclosure is not limited thereto.

Still another aspect of the present disclosure provides a cosmetic composition including the composition for transdermal delivery of the present disclosure.

The composition for transdermal delivery, *etc.* is as described above.

The cosmetic composition may be for skin improvement, or for disease prevention or improvement according to the physiological function of the bioactive material, but is not limited thereto.

In one embodiment, the cosmetic composition may be for skin improvement, but is not limited thereto.

In one embodiment, the cosmetic composition may be for skin regeneration, wound healing, anti-aging, wrinkle improvement, whitening, inflammation relief, antibacterial action, moisturizing, moisture increase, and antioxidant action, but is not limited thereto.

In one embodiment, the cosmetic composition may be for prevention or improvement of a skin disease, but is not limited thereto.

The skin disease refers to a disease in which abnormal symptoms such as inflammation, erythema, thickening, fibrosis, and dead skin cells appear on the skin, which are caused by genetic factors, physiological factors, and environmental factors. For example, the skin disease may be atopic dermatitis, allergy, psoriasis, seborrheic dermatitis, contact dermatitis, lupus erythematosus, acne, warts, viral infection, papular urticaria, *etc.,* but is not limited thereto.

As used herein, the "prevention" refers to any action whereby the onset of a disease (*e.g.*, skin disease) or progression thereof is restrained or retarded.

As used herein, the "improvement" refers to any action whereby parameters related to alleviation or treatment of conditions, *e.g.*, severity of symptoms are at least reduced. In the present disclosure, the cosmetic composition may be prepared in a preparation selected from the group consisting of solutions, ointments for external use, creams, foams, nourishing lotions, softening lotions, perfumes, packs, softening lotions, emulsions, makeup bases, essences, soaps, liquid cleansers, bath preparations, sunscreen creams, sun oils, suspensions, emulsions, pastes, gels, lotions, powder, soaps, surfactant-containing cleansing, oils, powder foundations, emulsion foundations, wax foundations, patches, and sprays, but is not limited thereto.

The cosmetic composition of the present disclosure may further include one or more cosmetically acceptable carriers which are blended in general skin cosmetics, and may be appropriately blended with, as common ingredients, for example, oil, water, surfactants, moisturizers, lower alcohols, thickeners, chelating agents, colorants, preservatives, perfumes, *etc.,* but is not limited thereto.

The cosmetically acceptable carriers included in the cosmetic composition of the present disclosure vary according to the formulation of the cosmetic composition.

When the formulation of the present disclosure is an ointment, a paste, a cream, or a gel, animal fats, vegetable oils, wax, paraffin, starch, tragacanth, cellulose derivative, polyethylene glycol, silicone, bentonite, silica, talc, zinc oxide, *etc.* may be used as a carrier component, but is not limited thereto. These may be used alone or in a mixture of two or more thereof.

When the formulation of the present disclosure is a powder or a spray, lactose, talc, silica, aluminum hydroxide, calcium silicate, polyamide powder, *etc.* may be used as a carrier component. Particularly, when the formulation is a spray, a propellant such as chlorofluorohydrocarbon, propane/butane or dimethyl ether may be additionally included, but is not limited thereto. These may be used alone or in a mixture of two or more thereof.

When the formulation of the present disclosure is a solution or an emulsion, a solvent, a solubilizer, or an emulsifier may be used as a carrier component. For example, water, glycerin, ethanol, isopropanol, ethyl carbonate, ethyl acetate, benzyl alcohol, benzyl benzoate, propylene glycol, 1,3-butyl glycol oil, *etc.* may be used. Particularly, cotton seed oil, peanut oil, corn seed oil, olive oil, castor oil and sesame oil, glycerol aliphatic ester, polyethylene glycol, or sorbitan fatty acid ester may be used, but is not limited thereto. These may be used alone or in a mixture of two or more thereof.

When the formulation of the present disclosure is a suspension, a liquid-phase diluent such as water, glycerin, ethanol or propylene glycol, a suspension such as ethoxylated isostearyl alcohol, polyoxyethylene sorbitol ester, polyoxyethylene sorbitan ester, microcrystalline cellulose, aluminum metahydroxide, bentonite, agar or tragacanth, *etc.* may be used as a carrier component, but is not limited thereto. These may be used alone or in a mixture of two or more thereof.

When the formulation of the present disclosure is a soap, fatty acid alkali metallic salts, fatty acid hemiester salts, fatty acid protein hydrolysates, isethionate, lanoline derivatives, aliphatic alcohols, vegetable oil, glycerol, sugar, *etc.* may be used as a carrier component, but is not limited thereto. These may be used alone or in a mixture of two or more thereof.

Still another aspect of the present disclosure provides a quasi-drug composition including the composition for transdermal delivery of the present disclosure.

The quasi-drug composition may be for skin improvement, or for prevention or improvement of a skin disease according to the physiological function of the bioactive material, but is not limited thereto.

The composition for transdermal delivery, bioactive material, skin improvement, skin disease, prevention, and improvement, *etc.* are as described above.

As used herein, the "quasi-drug" refers to an article having a milder action than drugs, among articles being used for the purpose of diagnosis, treatment, improvement, alleviation, handling, or prevention of human or animal diseases. For example, according to the Pharmaceutical Affairs Law, the quasi-drugs are those, excluding articles used as drugs, including articles used for the purpose of treating or preventing human or animal diseases, which have a mild action on or have no direct influence on the human body.

The quasi-drug composition of the present disclosure may be prepared in a formulation selected from the group consisting of body cleanser, foam, soap, mask, ointment, cream, lotion, essence, and spray, but is not limited thereto.

In the cosmetic composition or quasi-drug composition of the present disclosure, the skin-permeable peptide consisting of any one amino acid sequence of SEQ ID NO: 5 to SEQ ID NO: 8, a derivative thereof, or a fragment thereof may be included in an amount of 0.01% by weight to 100.0% by weight, 0.1% by weight to 10% by weight, based on the total amount of the composition.

Still another aspect of the present disclosure provides a pharmaceutical composition for external use on the skin, the pharmaceutical composition including the composition for transdermal delivery of the present disclosure.

The pharmaceutical composition may be for prevention or treatment of a skin disease according to the physiological function of the bioactive material, but is not limited thereto. The composition for transdermal delivery, preparation for external use on the skin, bioactive material, skin disease, and prevention, *etc.* are as described above.

As used herein, the "treatment" refers to any action whereby symptoms of a disease (*e.g.*, skin disease) have taken a turn for the better or been modified favorably. In the present disclosure, the treatment may include all actions to prevent recurrence of a disease, to alleviate symptoms, to reduce the rate of progression, to alleviate a disease state, to temporarily or continuously alleviate a disease state, to achieve remission, or to improve a prognosis.

The pharmaceutical composition of the present disclosure may include a bioactive material and a pharmaceutically acceptable carrier, in addition to the skin-permeable peptide consisting of any one amino acid sequence of SEQ ID NO: 5 to SEQ ID NO: 8, a derivative thereof, or a fragment thereof.

As used herein, the "pharmaceutically acceptable" means that it does not interfere with the biological activity and properties of the administered compound without stimulating organisms during administration, which may be commonly used in the pharmaceutical field. The pharmaceutical composition of the present disclosure may be formulated together with the carrier to be used as a food, medicine, feed additive, drinking water additive, *etc.*

The type of the carrier is not particularly limited, and any carrier commonly used in the art may be used. Non-limiting examples of the carrier may include saline, sterile water, Ringer's solution, buffered saline, albumin, lactose, dextrose, sucrose, sorbitol, mannitol, xylitol, erythritol, maltitol, maltodextrin, glycerol, ethanol, *etc.* These may be used alone or in a mixture of two or more thereof, but are not limited thereto.

In addition, the pharmaceutical composition of the present disclosure may be used by adding other pharmaceutically acceptable additives such as excipients, diluents, antioxidants, buffers, or bacteriostats, and by additionally adding fillers, extenders, wetting agents, disintegrants, dispersants, surfactants, binders or lubricants, *etc.,* as needed, but is not limited thereto.

The pharmaceutical composition of the present disclosure may be used after being formulated into various formulations suitable for external use on the skin.

Non-limiting examples of the formulations for external use on the skin may include aerosols, sprays, cleansers, ointments, coating powders, oils, creams, *etc.,* but are not limited thereto, as long as they may function as the formulations for external use on the skin.

In order to formulate the pharmaceutical composition of the present disclosure for external use on the skin, sterilized aqueous solutions, non-aqueous solvents, suspensions, emulsions, freeze-dried preparations, external preparations, *etc.* may be used. As the non-aqueous solvents and suspensions, propylene glycol and polyethylene glycol, vegetable oil such as olive oil, esters such as ethyl oleate, *etc.* may be used, but are not limited thereto.

Further, more specifically, when the pharmaceutical composition of the present disclosure is formulated, the pharmaceutical composition of the present disclosure is mixed in water with a stabilizer or buffer to prepare a solution or suspension, which may then be formulated in units such as ampoules. In addition, when the pharmaceutical composition of the present disclosure is formulated into an aerosol, a propellant, *etc.* may be blended with additives so that the water-dispersed concentrate or wet powder is dispersed, but is not limited thereto.

In addition, when the pharmaceutical composition of the present disclosure is formulated into an ointment, a cream, *etc.,* animal fats, vegetable oils, wax, paraffin, starch, tragacanth, cellulose derivative, polyethylene glycol, silicone, bentonite, silica, talc, zinc oxide, *etc.* may be used as a carrier component, but is not limited thereto.

When the pharmaceutical composition of the present disclosure is formulated for external use on the skin, it may have more preferably a formulation selected from the group consisting of a gel, a patch, a spray, an ointment, a plaster, a lotion, a liniment agent, a paste agent and a cataplasma, but is not limited thereto.

A pharmaceutically effective amount of the pharmaceutical composition of the present disclosure and the effective dosage for external use may vary depending on the formulation method of the pharmaceutical composition, administration method, administration time and/or administration route, *etc.,* and may vary depending on various factors including the type and degree of reaction to be achieved by administration of the pharmaceutical composition, a subject's type, age, body weight, general health conditions, disease symptom or severity, sex, diet, excretion, drugs used simultaneously or separately in the subject, and other components of the composition, and similar factors well known in the medical field, and those skilled in the art may easily determine and prescribe an effective dosage for the desired treatment. Administration of the pharmaceutical composition of the present disclosure may be made once a day or divided into several times. Therefore, the dosage is not intended to limit the scope of the present disclosure in any aspect.

In addition, administration in the present disclosure may be administration for external use, and a preferred dosage of the pharmaceutical composition of the present disclosure may be 1 mg/kg to 1,000 mg/kg per day.

The administration route and administration mode of the pharmaceutical composition of the present disclosure may be independent, and are not particularly limited in the mode, and the pharmaceutical composition may follow any administration route and administration mode, as long as it may reach the target site. The pharmaceutical composition may be administered in the manner of the external preparation for the skin. Specifically, a method of applying or spraying the composition to the disease site may be used, but is not limited thereto.

The pharmaceutical composition of the present disclosure may be preferably administered by applying or spraying, and specifically, it is administered by topical application to areas that need to improve skin conditions or to prevent, improve, or treat a skin disease.

Still another aspect of the present disclosure provides a kit including the composition for transdermal delivery of the present disclosure; and a bioactive material.

The kit may be for transdermal delivery, skin permeation, skin improvement, prevention or treatment of a skin disease, but is not limited thereto. The kit may be provided such that the composition for transdermal delivery and the bioactive material may be stored in separate containers and mixed at the time of use, but is not limited thereto.

In addition, the kit of the present disclosure may include a composition consisting of one or more other components, a solution, or a device for transdermal delivery, skin permeation, skin improvement, prevention or treatment of a skin disease, but is not limited thereto. For examples of other components, suitable carriers, solubilizers, buffers, stabilizers, *etc.* may be included, but are not limited thereto. The carriers may include soluble carriers or insoluble carriers, and examples of the soluble carriers may include a physiologically acceptable buffer known in the art, *e.g.*, PBS, and examples of the insoluble carriers may include polystyrene, polyethylene, polypropylene, and polyester, polyacrylonitrile, fluorine resins, crosslinked dextran, polysaccharides, polymers such as magnetic microparticles plated with metal on latex, other paper, glass, metals, agarose, and combinations thereof, but are not limited thereto.

In addition, the kit of the present disclosure may further include a user guide describing conditions for performing an optimal reaction. The guide is a printout that explains how to use the kit, *e.g.*, reaction conditions, *etc.* The guide includes a brochure in the form of a pamphlet or leaflet, a label affixed to the kit, and instructions on the surface of a package containing the kit. In addition, the guide includes information disclosed or provided through electronic media such as the Internet.

Still another aspect of the present disclosure provides a method of preparing the composition for transdermal delivery of the present disclosure.

Still another object of the present disclosure provides a transdermal delivery method of a physiologically active peptide, the method including applying, to the skin or mucous membrane, the composition for transdermal delivery of the present disclosure; and the bioactive material.

Still another object of the present disclosure provides a method of improving the skin, the method including applying, to the skin or mucous membrane, the composition for transdermal delivery of the present disclosure; and a bioactive material.

Still another object of the present disclosure provides a method of preventing or treating a skin or mucosal disease, the method including applying the pharmaceutical composition of the present disclosure to the skin or mucous membrane of a subject excluding humans.

Still another object of the present disclosure provides a method of preventing or improving a skin or mucosal disease, the method including applying, to the skin, the cosmetic composition or quasi-drug composition of the present disclosure.

Still another object of the present disclosure provides a method of preparing the cosmetic composition, quasi-drug composition, or pharmaceutical composition, the method including mixing the composition for transdermal delivery of the present disclosure and a bioactive material.

Still another object of the present disclosure provides use of the peptide consisting of any one amino acid sequence of SEQ ID NO: 5 to SEQ ID NO: 8, a derivative thereof, or a fragment thereof of the present disclosure for the skin permeation of a bioactive material.

Still another object of the present disclosure provides use of the peptide consisting of any one amino acid sequence of SEQ ID NO: 5 to SEQ ID NO: 8, a derivative thereof, or a fragment thereof of the present disclosure for the transdermal delivery of a bioactive material.

Still another object of the present disclosure provides use of the peptide consisting of any one amino acid sequence of SEQ ID NO: 5 to SEQ ID NO: 8, a derivative thereof, or a fragment thereof of the present disclosure and a bioactive material; a cosmetic composition including the same; or a quasi-drug composition for the skin improvement, or prevention or improvement of a skin disease.

Still another object of the present disclosure provides use of a pharmaceutical composition including the peptide consisting of any one amino acid sequence of SEQ ID NO: 5 to SEQ ID NO: 8, a derivative thereof, or a fragment thereof of the present disclosure and a bioactive material for the prevention or treatment of a skin disease. The composition for transdermal delivery, bioactive material, skin improvement, pharmaceutical composition, cosmetic composition, quasi-drug composition, skin disease, prevention, improvement, and treatment are as described above.

As used herein, the term "subject" refers to any animal including mice, livestock, and mammals including humans, *etc.,* but is not limited thereto.

As used herein, the term "applying" means bringing the composition for transdermal delivery of the present disclosure and a bioactive material into contact with a subject's skin by any suitable method, and includes all actions aimed at absorbing the composition for transdermal delivery of the present disclosure and a bioactive material into the skin, but is not limited thereto.

**[Forms for Implementation of the Invention]**

Hereinafter, the present disclosure will be described in more detail with reference to Examples. However, these Examples and Experimental Examples are only for illustrating the present disclosure, and the scope of the present disclosure is not intended to be limited by these Examples and Experimental Examples.

**Preparation Example 1: Preparation of skin-permeable peptide**

To examine function of skin-permeable peptides, peptides of SEQ ID NO: 5 to SEQ ID NO: 8 and derivative peptides thereof were prepared.

**1-1: Synthesis of peptide of SEQ ID NO: 1**

71.4 mg (0.10 mmol) of 2-chlorotrityl chloride resin (1.4 mmol/g loaded resin) purchased from Novabiochem Corporation was weighed, the resin was solvated with 7.5 mL of methyl chloride and reacted for 5 minutes, and then the methyl chloride was removed.

Thereafter, Fmoc-Lys(Boc)-OH (281.1 mg, 0.60 mmol) was completely dissolved in 7.5 mL of MC solvent, 0.21 mL, 1.2 mmol of *N,N'*-diisopropylethylamine (DIPEA) was added, and the solution was added to the resin. The reaction solution was subjected to shaking at room temperature for 12 hours, and then washed four times with 10 mL of DMF solvent.

Then, 7.5 mL of a 20% (w/v) piperidine DMF solution was added, followed by shaking for 15 minutes, and 9-fluorenylmethyloxycarbonyl (Fmoc) protecting groups were completely removed from the material which had been added to the above-described resin, and this was washed four times using 10 mL of DMF solvent (washing four times with 10 mL each). At this stage, the deprotection reaction of the Fmoc protecting groups was confirmed by a Kaiser test (E. Kaiser et al. Anal. Biochem., 1970, 34(2), 595-598.).

Meanwhile, Fmoc-Leu-OH (212.0 mg, 0.60 mmol) and hexafluorophosphate benzotriazole tetramethyluronium (HBTU) (227.6 mg, 0.60 mmol) and hydroxybenzotriazole (HOBt) (81.0 mg, 0.6 mmol) were completely dissolved in 7.5 mL of DMF solvent, and then DIPEA (0.21 mL, 1.2 mmol) was added thereto, and the mixture was added to the above resin. After shaking at room temperature for 3 hours, the reaction solution was washed four times each with 10 mL of DMF solvent. At this stage, the Kaiser test was performed to confirm the completion of the reaction.

Next, the peptides were continuously condensed (coupled) according to the same synthesis cycle as below.

(1) Washing with DMF solvent (10 mL) four times;
(2) Deprotecting using 20% (w/v) piperidine DMF solution (7.5 mL) for 15 minutes;
(3) Washing with DMF solvent (10 mL) four times;
(4) Adding Fmoc-amino acid, HBTU, HOBt, DIPEA;
(5) Activation of amino acids by adding HBTU, HOBt, and DIPEA condensation reagents and condensation for 3 hours;
(6) Washing with DMF solvent (10 mL) four times.

The above (1) to (6) were repeated, and at this time, Fmoc-protected amino acids (0.60 mmol) after Fmoc-Leu-OH were added to a resin reaction vessel in the order described below and condensed.

(i) Fmoc-Tyr(tBu)-OH;
(ii) Fmoc-Lys(Boc)-OH;
(iii) Fmoc-Leu-OH;
(iv) Fmoc-Ile-OH;
(v) Fmoc-Lys(Boc)-OH;
(vi) Fmoc-Lys(Boc)-OH;
(vii) Fmoc-Phe-OH;
(viii) Fmoc-Leu-OH;
(ix) Fmoc-Lys(Boc)-OH;
(x) Fmoc-Lys(Boc)-OH.

After (6) after Fmoc-Lys(Boc)-OH condensation, a 20% piperidine DMF solution (7.5 mL) was finally treated, followed by washing with 10 mL of each of DMF solvent three times, MC solvent three times, and diethyl ether solvent three times.

Immediately after the completion of the synthesis, the peptides were cleaved from the peptide-coupled resin using a mixture of trifluoroacetic acid/triisopropylsilane/water (95:2.5:2.5) (10 mL) for 3 hours. The mixed solution thus obtained was treated with 100 mL of a diethyl ether solvent stored in a refrigerator to produce a precipitate. The obtained precipitate was completely precipitated by centrifugation, and trifluoroacetic acid was primarily removed, and the above procedure (step of washing the precipitate by adding 100 mL of a diethyl ether solvent and centrifuging; a procedure of removing the trifluoroacetic acid, primary removal of which was attempted) was repeated twice to obtain a solidified precipitate.

The precipitate (peptide) was purified by HPLC using a 5% to 100% acetonitrile/water gradient solvent system containing 0.001% trifluoroacetic acid over 50 minutes using a C-18 column. The purified fraction was lyophilized to obtain 155 mg of a skin-permeable peptide of SEQ ID NO: 1 as a white powdery trifluoroacetate salt.

SEQ ID NO: 1: $H_2N$–[Lys–Lys–Leu–Phe–Lys–Lys–Ile–Leu–Lys–Tyr–Leu–Lys]–$CO_2H$

MS (ESI) *m/e,* [M+H]+=1550.17; (155 mg)

### 1-2: Synthesis of peptides of SEQ ID NO: 2 to SEQ ID NO: 8

The following peptides of SEQ ID NO: 2 to SEQ ID NO: 8 were prepared in the same preparation process as in Preparation Example 1-1, except that the order of Fmoc-protected amino acids was changed.

SEQ ID NO: 2: $H_2N$–[Arg–Arg–Leu–Phe–Arg–Arg–Ile–Leu–Arg–Tyr–Leu–Arg]–$CO_2H$

MS (ESI) *m/e,* [M+H]+=1718.23; (171 mg)

SEQ ID NO: 3: $H_2N$–[Arg–Phe–Leu–Phe–Arg–Leu–Ile–Leu–Arg–Tyr–Leu–Arg]–$CO_2H$

MS (ESI) *m/e,* [M+H]+=1666.18; (166 mg)

SEQ ID NO: 4: $H_2N$–[Arg–Arg–Leu–Phe–Arg–Leu–Ile–Leu–Arg–Tyr–Leu–Phe]–$CO_2H$

MS (ESI) *m/e,* [M+H]+=1666.18; (166 mg)

SEQ ID NO: 5: $H_2N$–[Arg–Lys–Leu–Phe–Lys–Arg–Ile–Leu–Arg–Tyr–Leu–Lys]–$CO_2H$

MS (ESI) *m/e,* [M+H]+=1634.2; (153 mg)

SEQ ID NO: 6: $H_2N$–[Lys–Arg–Leu–Phe–Arg–Lys–Ile–Leu–Lys–Tyr–Leu–Arg]–$CO_2H$

MS (ESI) *m/e,* [M+H]+=1634.2; (143 mg)

SEQ ID NO: 7: $H_2N$–[Lys–Arg–Leu–Phe–Lys–Arg–Ile–Leu–Arg–Tyr–Leu–Lys]–$CO_2H$

MS (ESI) *m/e,* [M+H]+=1634.2; (132 mg)

SEQ ID NO: 8: $H_2N$–[Arg–Lys–Leu–Phe–Arg–Lys–Ile–Leu–Lys–Tyr–Leu–Arg]–$CO_2H$

MS (ESI) *m/e,* [M+H]$^+$=1634.2; (172 mg)

## Example 1: Evaluation of skin permeability of SEQ ID NO: 5 to SEQ ID NO: 8 and derivatives thereof for bioactive materials

In order to confirm the function as a skin-permeable peptide, the skin permeability for lidocaine was evaluated in comparison with a known peptide. A mixture of skin-permeable peptides consisting of any one amino acid sequence selected from SEQ ID NO: 5 to SEQ ID NO: 8 and derivatives thereof of SEQ ID NO: 1 to SEQ ID NO: 4, or the known peptide and lidocaine, or lidocaine alone was treated to evaluate skin permeability.

In detail, to evaluate the skin permeability, real human skin was used and the skin permeability was measured by a Franz diffusion cell assay, and the permeation amount of lidocaine was measured by HPLC and then compared. The known peptide sequence is as follows.

- Known peptide sequence (TAT): GRKKRRQRRRPQ

## Franz diffusion cell assay

Human skin used in this experiment was purchased from UMC Science Co., Ltd., Korea, and phosphate-buffered saline (PBS, Gibco, USA) was purchased from Sigma-Aldrich.

In order to measure skin permeability of skin-permeable peptides, Franz diffusion cell assay was performed according to the OECD (2004, Paris) Guidance Document for the Conduct of Skin Absorption Studies and the Ministry of Food and Drug Safety (2009) guidelines for alternative method to animal test for cosmetics (*in vitro* skin absorption test method).

First, the skin area was set to 3 cm$^2$, and the stratum corneum layer of the skin was placed facing upward on a receptor chamber. Thereafter, the donor chamber was placed on the stratum corneum layer and clamped. A magnetic bar was inserted into a receptor at the bottom of the Franz diffusion cell, and a buffer was filled through a sampling arm. The buffer was PBS at pH 7.4 when the test substance was water-soluble, or ethanol:water = 1:1 when the test substance was oil-soluble.

The temperature of the receptor fluid was maintained constant at 32°C $\pm$ 1°C, and the humidity was maintained at 30-70%, followed by stabilization for 30 minutes. Thereafter, a stirrer was set at 600 rpm. The skin of each group was treated with 1 mL of the sample at a concentration of 2 mM. Thereafter, the aqueous solution was mixed well by maintaining the speed of the magnetic stirrer bar at 600 rpm, which was inserted into the receptor. 6 hours later, sampling was performed through a sampling arm of the receptor, and for quantification, STD (100 μM standard sample) and the sample were analyzed through HPLC, and the permeability was expressed as a percentage (%) as compared to STD.

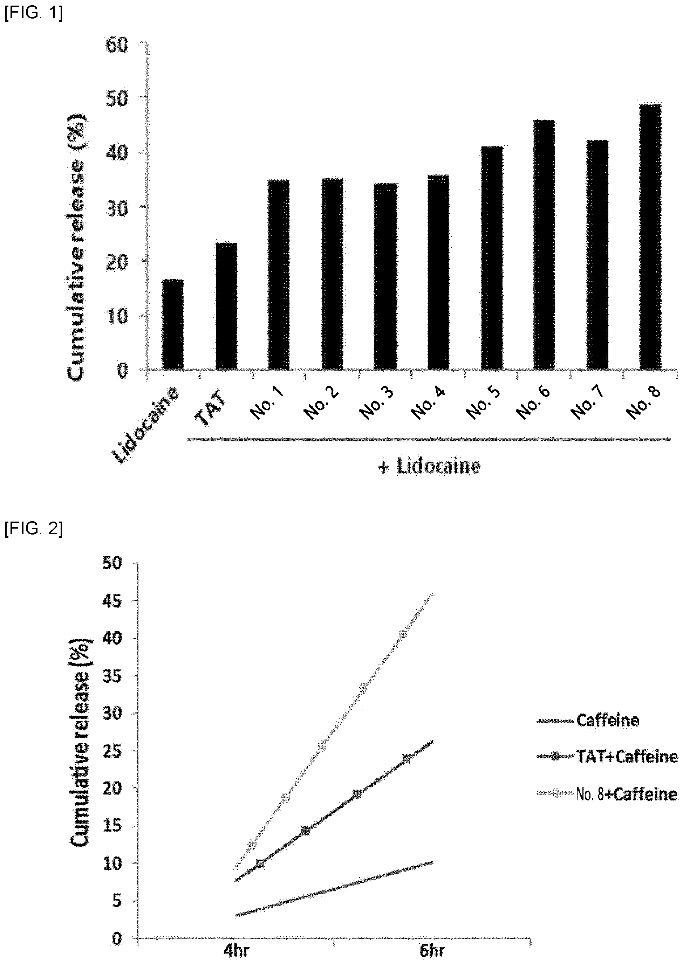

As a result, it was confirmed that when SEQ ID NOS: 5 to 8 and the derivatives thereof were used, the skin permeation amount of lidocaine was significantly superior to the case of using the known peptide and the case of not using the peptide (FIG. 1).

## Example 2: Evaluation of skin permeability of skin-permeable peptide for bioactive materials

Among the peptides of SEQ ID NO: 1 to SEQ ID NO: 8 described above, the skin permeability of the skin-permeable peptide of SEQ ID NO: 8 having the highest effect for caffeine was measured over time. A mixture of the skin-permeable peptide of SEQ ID NO: 8 and caffeine or caffeine alone was treated to evaluate skin permeability thereof.

In detail, Franz diffusion cell assay was performed using actual human skin as in Example 1, and the permeation amount of caffeine was measured by HPLC and then compared (FIG. 2).

As a result, it was confirmed that when SEQ ID NO: 8 was used, the skin permeation amount of caffeine was significantly superior to the case of using the known peptide and the case of not using the peptide.

## Example 3: Evaluation of skin permeability of skin-permeable peptide for bioactive materials having different molecular weights

In order to evaluate whether the skin-permeable peptides have skin permeability even for bioactive materials with different molecular weights, 3 kDa, 30 kDa, and 300 kDa FITC-conjugated collagen (COLA) and 800 kDa hyaluronic acid (HA) were treated after being mixed with the peptide of SEQ ID NO: 8, or treated alone, and aqueous solutions were obtained through Franz diffusion cell assay, and then FITC fluorescence values were analyzed using a fluorescence microplate reader (SpectraMax i3, Molecular Devices, USA).

The FITC conjugation method was performed in accordance with the manufacturer's instructions by purchasing an FITC conjugation kit (Lightning-Link® (ab188285)) from Abcam (Cambridge, MA, USA).

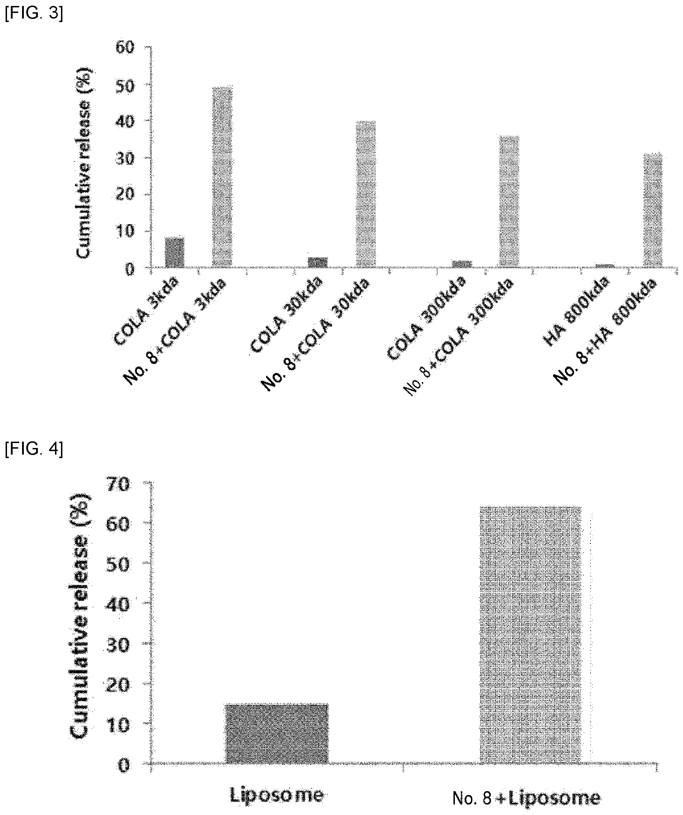

As a result, it was confirmed that when the mixture with the peptide of SEQ ID NO: 8 was used, strong skin permeability effects of collagens with different molecular weights (3 kDa, 30 kDa, and 300 kDa) and hyaluronic acid were observed (FIG. 3).

**Example 4: Evaluation of skin permeability of skin-permeable peptide for liposome**

In order to closely examine the effect of the skin-permeable peptides for the skin permeation effect of liposome, which is a type of physiologically active materialbioactive material, human skin was treated either with a mixture of the peptide of SEQ ID NO: 8 and liposome or liposome alone for 3 hours. Aqueous solutions were then obtained through Franz diffusion cell assay, and FITC fluorescence values were analyzed using a fluorescence microplate reader (SpectraMax i3, Molecular Devices, USA).

Fluorescein was liposomized using a liposome kit purchased from Sigma Aldrich (MO, USA) and liposomizing Fluorescein was used as liposome for the skin permeability tests. The sizes of liposomes were measured as 180 nm by SEM.

As a result, it was confirmed that when the mixture of the peptide of SEQ ID NO: 8 and liposome was treated, the skin permeation effect of the liposome was remarkably increased (FIG. 4).

**Example 5: Evaluation of efficacy improvement of bioactive material due to skin permeation effect of skin-permeable peptide for bioactive material**

In order to examine the efficacy improvement of the bioactive material due to the skin permeation effect of the active ingredient by the peptide of SEQ ID NO: 8, HDF cells were treated with a mixture of KTTKS peptide, which is a bioactive material, and the peptide of SEQ ID NO: 8, or treated with KTTKS peptide alone, and collagen production amounts were analyzed using an ELISA assay.

HDF cells used in this experiment were purchased from Thermo Fisher Scientific (Waltham, MA, USA). HDF cells were cultured in Dulbecco's Modified Eagle's Medium (DMEM) supplemented with 10% fetal bovine serum (FBS), 100 U/mL penicillin, and 100 $\mu$g/mL streptomycin (Gipco, Life Technologies, Grand Island, NY, USA) and cultured in a 5% $CO_2$ incubator at 37°C.

HDF cells at a density of $2 \times 10^5$ cells/mL were dispensed in a volume of 250 $\mu$L in 48 wells and allowed to stabilize for 24 hours. Samples were treated at different concentrations and cultured in a $CO_2$ incubator for 48 hours, and then each culture was obtained. In accordance with the manufacturer's instructions (Collagen type I ELISA Kit, R&D Biosystems), an antibody was diluted in a coating buffer, coated on micro wells, and incubated overnight at 4°C. After washing each well with a washing buffer three times, 100 $\mu$L of the culture was dispensed. The wells were left at room temperature for 1 hour, washed three times with a washing buffer, treated with 100 $\mu$L of avidin-HRP conjugated antibody, left at room temperature for 1 hour, and then washed three times again. 100 $\mu$L of TMB substrate was dispensed therein, left in the dark for 30 minutes, and treated with 50 $\mu$L of a stop solution, and then absorbance was measured at 450 nm with a microspectrophotometer (Molecular Device, Sunnyvale, CA, USA).

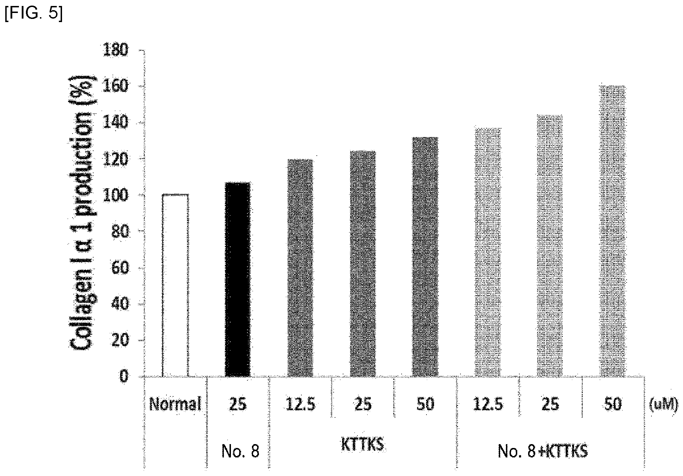

As a result, it was confirmed that when the mixture of the peptide of SEQ ID NO: 8 and KTTKS was treated, the production amount of collagen significantly increased in accordance with the increase in the skin permeation effect of the KTTKS peptide (FIG. 5).

**Example 6: Evaluation of human application test of efficacy improvement due to skin permeation effect of skin-permeable peptide for bioactive material**

In order to confirm the efficacy improvement due to the skin permeation effect of the active ingredient by the peptide of SEQ ID NO: 8 through a human application test, the human application test was conducted on 20 subjects by the SKINMED Clinical Trial Center. A test group was prescribed with a mixture of the peptide of SEQ ID NO: 8 and 1% ultra-low-molecular-weight hyaluronic acid, and a control group was prescribed with 1% ultra-low-molecular-weight hyaluronic acid without the peptide of SEQ ID NO: 8.

For the test subjects, the product application area was the forearm, the product application amount was about 0.5 g $\pm$ 0.1 g per time, and the product application cycle and method were twice per day (other life patterns were the same as usual). This test was conducted after the subjects had a skin rest for at least 30 minutes under constant temperature and humidity conditions (room temperature of 20-25°C, humidity of 40-60%) without air movement and direct sunlight. In order to accurately determine the effect of the present disclosure, the application area was prevented from touching water or sweating during the test period so that the product application area could be preserved as much as possible. In addition, in order to minimize the measurement errors, each measurement was repeated three times by the same tester, and the mean value was evaluated.

To examine whether or not the skin texture was improved, Visioscan was used to uniformly distribute light to the reflector, and images were taken with a video sensor chip and an objective lens. A non-glossy image of the skin surface (approximately 10 mm × 8 mm in size) represented by 255 gray levels was derived, and the SEr (Roughness) value representing the roughness of the skin was reflected among the derived measured values. A predetermined area was measured, and with comparison to the area before use of the product, the same area was measured again 24 hours after use of the product to thereby examine the skin roughness. As SEr increases, the skin roughness decreases, indicating that the skin texture was improved.

Whether or not the skin elasticity was improved was measured using a Cutometer dual MPA 580. The measuring principle of the Cutometer is based on a suction method, in which negative pressure deforms the skin mechanically. The pressure is created in the device and draws the skin into the aperture of the probe after a defined time. Inside the probe, the penetration depth is determined via a non-contact optical measuring system. This optical measuring system consists of a light source and a light receptor, as well as two prisms facing each other, which project the light from the transmitter to the receptor. The light intensity varies due to the penetration depth of the skin. The resistance of the skin to the negative pressure (firmness) and its ability to return to its original position (elasticity) are displayed as curves (in mm/time) in real time during the measurement. From these curves, as measurement parameters, R2 values are calculated to be consistent with elastic and visco-elastic properties of skin surface and skin aging. The closer the value is to 1 (100%), the more elastic it is. In this human application test, 24 hours after use of the product, the value of R2 (R2 = $U_a/U_f$; proportion of the maximum amplitudes) representing the skin elasticity of the test area was measured.

The gloss of the forearm area was measured using a SkinGlossMeter (SGM, Delfin). When the light from a 635 nm red diode laser is reflected from the skin surface, the amount of reflection is detected. The light passing through the diffracting microstructure inside the device was used to calculate the gloss value as an SGU (Skin Gloss Unit) parameter. The higher the gloss of the skin, the higher the measured value. In this human application test, the SGU values representing the skin gloss of the test area were measured before use of the product and 24 hours after use of the product.

In addition, the improvement rate was calculated by the following formula.

$$\text{Improvement rate (\%) formula} = \{(\text{before use} - \text{after use}) / \text{before use}\} \times 100$$

As a result, it was found that, after 24 hours, the test group showed an about 40-fold, 4-fold, and 5-fold increase in the average improvement rates of the skin texture, skin elasticity, and gloss, respectively, as compared to the control group (Table 1).

[Table 1]

| Efficacy | Average improvement rate 24 hrs after use of product | |
|---|---|---|
| | Control group | Test group |
| Skin texture (SEr value) | 1.0872% | 43.0538% |
| Skin elasticity (R2 value) | 3.4389% | 12.3372% |
| Gloss (SGU value) | 1.6963% | 8.3013% |

The above results confirmed that the skin-permeable peptide, derivative thereof, or fragment thereof of the present disclosure has excellent skin permeability, and therefore, it has a remarkable effect as a skin-permeable peptide.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds, are therefore intended to be embraced by the claims.

**Claims**

1. A composition for transdermal delivery of a bioactive material, the composition comprising a skin-permeable peptide consisting of any one amino acid sequence of SEQ ID NO: 5 to SEQ ID NO: 8, a derivative thereof, or a fragment thereof.

2. The composition for transdermal delivery of a bioactive material of claim 1, wherein the derivative has a substitution of an amino acid corresponding to any one position selected from the group consisting of positions 1, 2, 5, 6, 9, and 12 from the N-terminus of the skin-permeable peptide consisting of any one amino acid sequence of SEQ ID NO: 5 to SEQ ID NO: 8 with another basic amino acid.

3. The composition for transdermal delivery of a bioactive material of claim 2, wherein the basic amino acid is lysine or arginine.

4. The composition for transdermal delivery of a bioactive material of claim 1, wherein the derivative is any one or more selected from the group consisting of SEQ ID NO: 1 to SEQ ID NO: 4.

5. The composition for transdermal delivery of a bioactive material of claim 1, wherein the composition for transdermal delivery increases permeability of a bioactive material to any one or more selected from the group consisting of the skin and mucous membrane.

6. The composition for transdermal delivery of a bioactive material of claim 1, further comprising a carrier.

7. The composition for transdermal delivery of a bioactive material of claim 6, wherein the carrier is a liposome.

8. The composition for transdermal delivery of a bioactive material of claim 1, wherein the bioactive material is a material having a physiological action in the body.

9. A composition for skin-permeation of a bioactive material, the composition comprising a skin-permeable peptide consisting of any one amino acid sequence of SEQ ID NO: 5 to SEQ ID NO: 8, a derivative thereof, or a fragment thereof.

10. A carrier for transdermal delivery of a bioactive material, the carrier comprising a skin-permeable peptide consisting of any one amino acid sequence of SEQ ID NO: 5 to SEQ ID NO: 8, a derivative thereof, or a fragment thereof.

11. A skin-permeable carrier for a bioactive material, the carrier comprising a skin-permeable peptide consisting of any one amino acid sequence of SEQ ID NO: 5 to SEQ ID NO: 8, a derivative thereof, or a fragment thereof.

12. A cosmetic composition comprising the composition for transdermal delivery of claim 1.

13. A quasi-drug composition comprising the composition for transdermal delivery of claim 1.

14. A pharmaceutical composition for external use on the skin or mucous membrane, the pharmaceutical composition comprising the composition for transdermal delivery of claim 1.

15. A kit comprising the composition for transdermal delivery of claim 1; and a bioactive material.

16. A method of preparing the composition for transdermal delivery of claim 1.

17. A method of preparing a cosmetic composition, a quasi-drug composition, or a pharmaceutical composition, the method comprising mixing the composition for transdermal delivery of claim 1 and a bioactive material.

18. A transdermal delivery method of a bioactive material, comprising applying the composition for transdermal delivery of claim 1 and a bioactive material to the skin or mucous membrane.

19. A method of improving the skin, comprising applying the composition for transdermal delivery of claim 1 and a bioactive material to the skin or mucous membrane.

20. Use of a peptide consisting of any one amino acid sequence of SEQ ID NO: 5 to SEQ ID NO: 8, a derivative thereof, or a fragment thereof for the skin permeation of a bioactive material.

21. Use of a peptide consisting of any one amino acid sequence of SEQ ID NO: 5 to SEQ ID NO: 8, a derivative thereof, or a fragment thereof for the transdermal delivery of a bioactive material.

[FIG. 1]

[FIG. 2]

[FIG. 3]

[FIG. 4]

[FIG. 5]

## INTERNATIONAL SEARCH REPORT

| | International application No. |
|---|---|
| | **PCT/KR2022/004657** |

### A. CLASSIFICATION OF SUBJECT MATTER

**A61K 8/64**(2006.01)i; **A61K 8/14**(2006.01)i; **A61Q 19/00**(2006.01)i; **A61K 9/00**(2006.01)i; **A61K 47/42**(2006.01)i; **A61K 9/127**(2006.01)i; **C07K 7/08**(2006.01)i

According to International Patent Classification (IPC) or to both national classification and IPC

### B. FIELDS SEARCHED

Minimum documentation searched (classification system followed by classification symbols)

A61K 8/64(2006.01); A61K 38/08(2006.01); A61K 38/10(2006.01); A61K 47/42(2006.01); C07K 14/00(2006.01); C07K 7/06(2006.01); C07K 7/08(2006.01); C12N 15/82(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 피부투과성 펩타이드(cell permeable peptide), 경피 전달용 펩타이드(transdermal delivery peptides), 리포좀(liposome), 생리활성물질(bioactive substance), 피부(skin)

### C. DOCUMENTS CONSIDERED TO BE RELEVANT

| Category* | Citation of document, with indication, where appropriate, of the relevant passages | Relevant to claim No. |
|---|---|---|
| A | KR 10-2013-0070607 A (PROCELL THERAPEUTICS, INC.) 27 June 2013 (2013-06-27)<br>See entire document. | 1-21 |
| A | KR 10-1327555 B1 (SEOUL NATIONAL UNIVERSITY R&DB FOUNDATION) 08 November 2013 (2013-11-08)<br>See entire document. | 1-21 |
| A | KR 10-2016-0066284 A (PEPTRON, INC.) 10 June 2016 (2016-06-10)<br>See entire document. | 1-21 |
| A | US 2020-0318125 A1 (RIKEN) 08 October 2020 (2020-10-08)<br>See entire document. | 1-21 |
| A | KR 10-2017-0055920 A (PEPTRON, INC.) 22 May 2017 (2017-05-22)<br>See entire document. | 1-21 |

✓ Further documents are listed in the continuation of Box C.   ✓ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **14 December 2022** | **15 December 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2022/004657** |

| C. | DOCUMENTS CONSIDERED TO BE RELEVANT | |
| --- | --- | --- |
| Category* | Citation of document, with indication, where appropriate, of the relevant passages | Relevant to claim No. |
| A | JP 2020-527164 A (RIJKSUNIVERSITEIT GRONINGEN) 03 September 2020 (2020-09-03) See entire document. | 1-21 |

Form PCT/ISA/210 (second sheet) (July 2019)

**INTERNATIONAL SEARCH REPORT**

| International application No. |
| --- |
| **PCT/KR2022/004657** |

| Box No. I | Nucleotide and/or amino acid sequence(s) (Continuation of item 1.c of the first sheet) |

1.  With regard to any nucleotide and/or amino acid sequence disclosed in the international application, the international search was carried out on the basis of a sequence listing:

 a. ☑ forming part of the international application as filed:

  ☑ in the form of an Annex C/ST.25 text file.

  ☐ on paper or in the form of an image file.

 b. ☐ furnished together with the international application under PCT Rule 13*ter*.1(a) for the purposes of international search only in the form of an Annex C/ST.25 text file.

 c. ☐ furnished subsequent to the international filing date for the purposes of international search only:

  ☐ in the form of an Annex C/ST.25 text file (Rule 13*ter*.1(a)).

  ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

**INTERNATIONAL SEARCH REPORT**
Information on patent family members

International application No.

**PCT/KR2022/004657**

| Patent document cited in search report | | | Publication date (day/month/year) | Patent family member(s) | | | Publication date (day/month/year) |
|---|---|---|---|---|---|---|---|
| KR | 10-2013-0070607 | A | 27 June 2013 | KR | 10-1393397 | B1 | 14 May 2014 |
| | | | | WO | 2013-077681 | A1 | 30 May 2013 |
| KR | 10-1327555 | B1 | 08 November 2013 | KR | 10-2012-0104036 | A | 20 September 2012 |
| KR | 10-2016-0066284 | A | 10 June 2016 | KR | 10-1732124 | B1 | 02 May 2017 |
| US | 2020-0318125 | A1 | 08 October 2020 | JP | 6888784 | B2 | 16 June 2021 |
| | | | | US | 11279941 | B2 | 22 March 2022 |
| | | | | WO | 2017-126604 | A1 | 22 November 2018 |
| | | | | WO | 2017-126604 | A1 | 27 July 2017 |
| KR | 10-2017-0055920 | A | 22 May 2017 | KR | 10-1782569 | B1 | 28 September 2017 |
| | | | | KR | 10-2017-0101173 | A | 05 September 2017 |
| | | | | WO | 2017-082692 | A1 | 18 May 2017 |
| JP | 2020-527164 | A | 03 September 2020 | EP | 3638371 | A2 | 22 April 2020 |
| | | | | US | 2020-0368315 | A1 | 26 November 2020 |
| | | | | WO | 2018-231058 | A2 | 20 December 2018 |
| | | | | WO | 2018-231058 | A3 | 07 February 2019 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 102274435

**Non-patent literature cited in the description**

- **E. KAISER et al.** *Anal. Biochem.,* 1970, vol. 34 (2), 595-598